Europäisches Patentamt

European Patent Office

Office européen des brevets

(19)

(11) Veröffentlichungsnummer: **0 191 395**
**B1**

(12) **EUROPÄISCHE PATENTSCHRIFT**

(45) Veröffentlichungstag der Patentschrift:
**26.07.89**

(51) Int. Cl.⁴: **C 07 D 307/60**

(21) Anmeldenummer: **86101378.7**

(22) Anmeldetag: **03.02.86**

(54) Verfahren zum Reinigen von rohem Maleinsäureanhydrid.

(30) Priorität: **07.02.85 DE 3504146**

(43) Veröffentlichungstag der Anmeldung:
**20.08.86 Patentblatt 86/34**

(45) Bekanntmachung des Hinweises auf die Patenterteilung:
**26.07.89 Patentblatt 89/30**

(84) Benannte Vertragsstaaten:
**AT BE DE FR GB IT**

(56) Entgegenhaltungen:
**DE-A- 1 021 356**
**DE-A- 2 356 049**

(73) Patentinhaber: **BASF Aktiengesellschaft,
Carl-Bosch-Strasse 38, D-6700 Ludwigshafen (DE)**

(72) Erfinder: **Zimmerling, Dieter, Brüsseler Ring 43,
D-6700 Ludwigshafen (DE)**
Erfinder: **Schmidt, Johannes E., Dr., Pariser Strasse 23,
D-6700 Ludwigshafen (DE)**
Erfinder: **Seubert, Rolf, Dr., Bensheimer Ring 23a,
D-6710 Frankenthal (DE)**
Erfinder: **Fischer, Karl, Dr., Bahnhofstrasse 49,
D-6521 Hohen-Sülzen (DE)**
Erfinder: **Sauer, Friedrich, Karlbacher Weg 24,
D-6719 Obersülzen (DE)**

## Beschreibung

Diese Erfindung betrifft ein Verfahren zur Gewinnung von hochreinem Maleinsäureanhydrid durch fraktionierte Destillation von rohem Maleinsäureanhydrid.

Maleinsäureanhydrid wird bekanntlich durch katalytische Dampfphasenoxidation von Benzol, Naphthenen, Butenen und Butan hergestellt. Ausserdem gewinnt man es bei der Herstellung von Phthalsäureanhydrid durch Oxidation von o-Xylol oder Naphthalin als Nebenprodukt. Man isoliert es aus dem rohen Maleinsäureanhyrid, üblicherweise durch fraktionierte Destillation, bei der es als weisses, praktisch reines Maleinsäureanhydrid gewonnen wird.

Es hat sich jedoch gezeigt, dass das so erhaltene weisse Maleinsäureanhydrid bei längerem Stehen zu Verfärbungen neigt. Die Verfärbung tritt noch schneller ein, wenn man das Maleinsäureanhydrid in geschmolzenem Zustand hält. Da derartige Verfärbungen bei der weiteren Verwendung des Maleinsäureanhydrids mitunter erheblich stören, vor allem beim Einsatz für die Herstellung von z.B. Polyesterharzen und Alkydharzen, wurde u.a. schon vorgeschlagen, nicht farbstabiles Maleinsäureanhydrid durch eine chemische Behandlung in hochreines und farbstabiles Maleinsäureanhydrid zu überführen. So gibt man nach den Angaben der GB-PS 1 204 846 z.B. Dibenzylsulfid zum destillierten Maleinsäureanhydrid. Aus der DE-OS 20 08 619 ist bekannt, dass man farbbeständige Ware erhält, wenn man flüssiges Maleinsäureanhydrid über eine Füllung bestimmter anorganischer Salze, wie Bariumchlorid leitet.

Die Behandlung von Maleinsäureanhydrid mit chemischen Zusätzen ist technisch aufwendig und hat ausserdem den Nachteil, dass weitere Verunreinigungen in das Maleinsäureanhydrid eingebracht werden. Deshalb wurde nach einem Verfahren gesucht, das es gestattet, hochreines und farbstabiles Maleinsäureanhydrid ohne chemische Behandlung zu erhalten.

Es wurde nun gefunden, dass man bei der Herstellung von Maleinsäureanhydrid durch fraktionierte Destillation von rohem Maleinsäureanhydrid ein hochreines, farbstabiles und lagerbeständiges Maleinsäureanhydrid erhält, wenn man das von Leichtsiedern und Schwersiedern abgetrennte dampfförmige Maleinsäureanhydrid, welches einen Maleinsäuregehalt von mindestens 98 Gew.% aufweist, partiell so kondensiert, dass 0,5 bis 15 Gew.% des dampfförmigen Maleinsäureanhydrids aus der Dampfphase als flüssiges Vorkondensat abgeschieden werden und das restliche dampfförmige hochreine Maleinsäureanhydrid anschliessend total kondensiert wird.

Nach dem neuen Verfahren geht man von einem Maleinsäureanhydrid aus, das auf bekannte Weise, z.B. durch katalytische Oxidation von Benzol, Naphthenen, Phenol, Furan, Buten-1, Buten-2, Butan, o-Xylol oder Naphthalin erhalten wurde. Das rohe Maleinsäureanhydrid, welches man bei einer dieser Synthesen gewinnt, wird einer an sich bekannten fraktionierten Destillation unterworfen, die sowohl kontinuierlich als auch diskontinuierlich sein kann.

Bei dieser Reindestillation werden vom rohen Maleinsäureanhydrid Leichtsieder und Schwersieder abgetrennt, während man das zu isolierende Maleinsäureanhydrid als dampfförmige Fraktion abnimmt. Diese Fraktion hat einen Maleinsäureanhydridgehalt von mindestens 98 Gew.%. Der erfindungsgemässe Schritt besteht nun darin, dass man diese dampfförmige Fraktion, die weitgehend reines, aber nicht farbstabiles Maleinsäureanhydrid enthält, partiell so kondensiert, dass zuerst 0,5 bis 15 Gew.%, vorzugsweise 0,5 bis 10 Gew.% der dampfförmigen Fraktion flüssig abgeschieden werden, während man die Restdampfmenge als hochreines und farbstabiles Maleinsäureanhydrid kondensiert.

Die Teilkondensation der dampfförmigen Fraktion nimmt man z.B. dadurch vor, dass man den dampfförmigen Strom durch einen Kühler leitet, wobei die Temperatur so geregelt wird, dass die gewünschte Teilmenge des dampfförmigen Maleinsäureanhydrids kondensiert und damit als Vorkondensat vom Dampfstrom flüssig abgetrennt werden kann. Der Dampfstrom, der nach Abtrennung des Vorkondensates aus reinstem und farbstabilem Maleinsäureanhydrid besteht, wird dann wie üblich total kondensiert.

Das erfindungsgemässe Verfahren kann sich an die übliche Destillation von rohem Maleinsäureanhydrid anschliessen.

Nach einer kontinuierlichen Arbeitsweise verfährt man z.B. so, dass man ein rohes Maleinsäureanhydrid, das z. B. bis zu 25 Gew. % an Verunreinigungen, wie Maleinsäure, Citraconsäure, Phthalsäure oder deren Anhydride sowie Benzoesäure und sonstige Verunreinigungen enthält, in einer Destillationskolonne, die mit Böden ausgestattet ist, so destilliert, dass man Leichtsieder über Kopf und Schwersieder als Sumpf abtrennt. Im Seitenabzug der Kolonne wird das auf diese Weise von den Nebenprodukten weitgehend befreite Maleinsäureanhydrid dampfförmig entnommen. Es hat eine Reinheit von mindestens 98 Gew.%. Zur Teilkondensation leitet an dieses dampfförmige Maleinsäureanhydrid z.B. durch eine Kühlschlange, in der man durch Temperaturregulierung des Kühlmediums die Teilkondensation so steuert, dass die erforderliche Menge an Vorkondensat aus dem dampfförmigen Maleinsäureanhydrid abgetrennt werden kann. Das restliche dampfförmige Maleinsäureanhydrid wird nach dieser Teilkondensation durch Totalkondensation erhalten.

Man erhält nach dem erfindungsgemässen Verfahren ein hochreines farbstabiles Maleinsäureanhydrid mit einer Reinheit von über 98 Gew.% und einer Hitzefarbzahl von unter 40 Hazen. Die hohe Lagerstabilität des so hergestellten flüssig gelagerten Maleinsäureanhydrids äussert sich darin, dass sich die Hitzefarbzahl selbst nach einer Lagerzeit von 6 bis 8 Wochen nicht verändert.

Es ist überraschend, dass es auf einfache Weise gelingt, durch Abtrennung eines so geringen Anteils des durch fraktionierte Destillation erhaltenen Maleinsäureanhydrids durch Teilkondensation die für das lästige Nachdunkeln der Reinware verantwortlichen Nebenprodukte so weitgehend zu entfernen, dass dabei ein Maleinsäureanhydrid dieser hohen Qualität erhalten wird.

Beispiel

623 g/h eines rohen Maleinsäureanhydrids der Zusammensetzung 81 Gew.% Maleinsäureanhydrid, 6 Gew.% Maleinsäure, 4 Gew.% Citraconsäure, 4 Gew.% Phthalsäure, 4 Gew.% Benzoesäure und 1 Gew.% sonstige Verunreinigungen wurden mit einer Zulauftemperatur von 100 °C kontinuierlich auf den 18. Glockenboden einer Glaskolonne gegeben. Die Kolonne mit einem Durchmesser von 80 mm enthielt 60 Glockenböden. Sie wurde bei einem Druck von 150 mbar am Kopf betrieben.

Am Kolonnenkopf wurde eine Gesamtdestillatmenge von 1928 g/h (Kondensationstemperatur 138 °C) gemessen, wobei 10 g/h als Kopfabzug entnommen und 1918 g/h als Rücklauf auf den Kolonnenkopf gegeben wurden. Der Kopfabzug setzte sich wie folgt zusammen. Citraconsäureanhydrid 271 Gew.-ppm, Maleinsäureanhydrid 99,97 Gew.%. Eine Wasseranalyse des Kopfabzuges wurde nicht durchgeführt.

Am Kolonnensumpf wurden 100 g/h (Temperatur 163 °C) abgezogen. Der Sumpfaustrag enthielt 19 Gew.% Maleinsäureanhydrid, 25 Gew.% Benzoesäure, 25 Gew.% Phthalsäureanhydrid und sonstige Verunreinigungen, wie hochsiedende Kohlenwasserstoffe, hochmolekulare organische Säuren und Zersetzungsprodukte. Am 50. Glockenboden wurden 503 g/h dampfförmiges Maleinsäureanhydrid bei einer Temperatur von 139 °C abgezogen. Die Zusammensetzung betrug: 99,7 Gew.% Maleinsäureanhydrid, 0,3 Gew.% Citraconsäureanhydrid. Das dampfförmige Maleinsäureanhydrid wurde in einen kleinen stehenden Glaskühler geleitet. Als Kühlmedium wurde Marlotherm verwendet. Die Temperatur des Kühlmediums wurde so eingestellt, dass 2% der Gasmenge entsprechend 10 g/h als Vorkondensat partiell kondensiert wurden. Die Kondensationstemperatur lag bei 138,5 °C. Die restlichen 503 g/h dampfförmiges Maleinsäureanhydrid wurden in einem sich anschliessenden Glaskühler totalkondensiert. Nach der Totalkondensation betrug die Temperatur des Maleinsäureanhydrids noch 75 °C.

Sowohl das als Vorkondensat erhaltene Maleinsäurehydrid als auch das als Kondesat der Totalkondensation erhaltene reine Maleinsäureanhydrid wurde einer Hitzefarbzahl-Bestimmung unterworfen. Dabei ergaben sich die folgenden Werte:

| Hitzefarbzahl (Hazen-Einheiten) nach einer Lagerzeit von | | | | | |
|---|---|---|---|---|---|
| | 0 | 2 Wochen | 4 Wochen | 6 Wochen | 8 Wochen |
| 1. Vorkondensat | > 200 | > 200 | > 200 | > 200 | > 200 |
| 2. Kondensat   2 Gew.% | < 40 | < 40 | < 40 | < 40 | < 40 |
| 3. Kondensat   6 Gew.% | < 40 | < 40 | < 40 | < 40 | < 40 |
| 4. Kondensat 10 Gew.% | < 40 | < 40 | < 40 | < 40 | < 40 |

Bei den Versuchen 2 bis 4 wurde das reine Maleinsäureanhydrid untersucht, das nach Abtrennung von 2 bis 10 Gew.% Vorkondensat, bezogen auf das dem Seitenabzug der Kolonne entnommene dampfförmige Maleinsäureanhydrid, durch Totalkondensation erhalten wurde. Zur Ermittlung der sogen. Hitzefarbzahl oder Hitzestabilität wurde die Probe im Reagenzglas 2 Stunden auf 140 °C erhitzt und dann mit der Hazen-Skala verglichen.

## Patentansprüche

1. Verfahren zur Gewinnung von hochreinem, farbstabilen Maleinsäureanhydrid durch fraktionierte Destillation von rohem Maleinsäureanhydrid, dadurch gekennzeichnet, dass man das von Leichtsiedern und Schwersiedern abgetrennte dampfförmige Maleinsäureanhydrid, welches einen Maleinsäuregehalt von mindestens 98 Gew.% aufweist, partiell so kondensiert, dass 0,5 bis 15 Gew.% des dampfförmigen Maleinsäureanhydrids aus der Dampfphase als flüssiges Vorkondensat abgeschieden werden und das restliche dampfförmige hochreine Maleinsäureanhydrid anschliessend total kondensiert wird.

2. Verfahren nach Anspruch 1, dadurch gekennzeichnet, dass man 0,5 bis 10 Gew.% des dampfförmigen Maleinsäureanhydrids als flüssiges Vorkondensat abtrennt.

## Revendications

1. Procédé d'obtention d'anhydride maléique de couleur stable et de pureté élevée par distillation fractionnée d'anhydride maléique brut, caractérisé en ce qu'on condense partiellement l'anhydride maléique à l'état de vapeur séparé des produits de bas point d'ébullition et des produits de haut point d'ébullition, anhydride maléique qui présente une teneur en acide maléique d'au moins 98% en poids, de façon à séparer de la phase vapeur de 0,5 à 15% en poids de l'anhydride maléique à l'état de vapeur en tant que pré-condensat liquide et l'anhydride maléique de pureté élevée à l'état de vapeur restant sera ensuite condensé totalement.

2. Procédé selon la revendication 1, caractérisé en ce qu'on sépare de 0,5 à 10% en poids de l'anhydride maléique à l'état de vapeur en tant que pré-condensat liquide.

## Claims

1. A process for obtaining very pure maleic anhydride having a stable color by fractional distillation of crude maleic anhydride, wherein the maleic anhydride vapor which has been separated off from low boilers and high boilers and contains not less than 98% by weight of maleic anhydride is partially condensed so that from 0,5 to 15% by weight of the maleic anhydride vapor is separated off from the vapor phase as a liquid precondensate, and the remaining very pure maleic anhydride vapor is then completely condensed.

2. A process as claimed in claim 1, wherein from 0.5 to 10% by weight of the maleic anhydride vapor is separated off as a liquid precondensate.